# EUROPEAN PATENT SPECIFICATION

(11) **EP 1 158 963 B1**
(45) Date of publication and mention of the grant of the patent: **22.01.2003**
(21) Application number: 00910723.6
(22) Date of filing: 28.02.2000
(51) Int. Cl.: A61K 9/26, A61K 31/4402, A61P 1/08

(54) **CONTROLLED-RELEASE COMPOSITIONS OF BETAHISTINE**
BETAHISTIN-HÄLTIGE FORMULIERUNG MIT KONTROLLIERTER WIRKSTOFFFREISETZUNG
COMPOSITIONS DE BETAHISTINE A LIBERATION PROLONGEE

(30) Priority: 05.03.1999 IT MI990454
(43) Date of publication of application: 05.12.2001
(73) Proprietor: FARMACEUTICI FORMENTI S.p.A., I-20149 Milan (IT)
(72) Inventor: FABIANI, Flavio, I-21040 Origgio (IT); FRIMONTI, Enrico, I-21040 Origgio (IT); VALENTI, Mauro, I-21040 Origgio (IT)
(74) Representative: Minoja, Fabrizio, Dr.
(86) International application number: EP0001642
(87) International publication number: WO00053162

(56) References cited:
- WO-A-98/18610
- FR-A- 2 432 313
- GB-A- 2 280 604

## Description

The present invention relates to the field of pharmaceutical technology.

More particularly, the invention relates to a novel controlled-release formulation of the active ingredient betahistine or of the pharmaceutically acceptable salts thereof.

Betahistine, or N-methyl-2-pyridineethanamine or 2-[2-(methylamino)ethyl]pyridine or [2-(2-pyridyl)ethyl]methylamine, and the respective salts: hydrochloride, dihydrochloride, methanesulfonate, fumarate and those listed in IT 1,229,237 and EP 0,397,025 patents, is a vasodilator active through the oral route used in the therapy of vertigo.

Betahistine has been commercially available for many years only in the form of prompt-release tablets or drops. No controlled-release pharmaceutical forms of Betahistine or any of the above mentioned salts thereof can be found in literature.

At present the posology of betahistine comprises 2-4 daily administrations, depending on the dosage of the concerned pharmaceutical form, and the total amount of active ingredient pro die is of 32 mg.

The preparation of a pharmaceutical form with a suitable release profile of Betahistine is desired, in that it would reduce the number of daily administrations to only one, while keeping the concentration of active ingredient steadily within the therapeutical dosage range.

It has now been found that controlled-release dosage forms of Betahistine can be prepared effectively and advantageously using a mixture of one or more hydrophilic or inert polymers capable of adsorbing water, the active ingredient and a lipophilic fatty compound.

Therefore the present provides controlled-release tablets comprising:
a) an active ingredient consisting of betahistine or a pharmaceutically acceptable salt thereof, incorporated in at least one fatty compound;
b) at least one hydrophilic polymer capable of adsorbing water;
c) suitable excipients, which tablet is obtainable as defined in claim 1.

The fatty compound consists of hydrophobic compounds with high molecular weight selected from the group consisting of fatty acids, long-chain fatty acid triglycerids, waxes, vegetable or mineral oils, high molecular alcohols or glycols, the esters and ethers thereof. The use of compounds with melting point ranging from 30 to 140 °C is preferred.

Examples of suitable hydrophilic polymers comprise acrylic acid polymers or co-polymers, polyethylene glycols, alginates, cellulose and derivatives (ethers, esters and salts).

Hydroxypropyl cellulose is particularly preferred.

The formulation can moreover be added with conventional excipients used commonly in the preparation of oral solid pharmaceutical forms.

Examples of these excipients comprise lubricants, diluents, coloring agents.

Each tablet typically contains an amount of active ingredient of 4 - 100 mg, preferably 8 - 64 mg of betahistine dihydrochloride. Particularly preferred are tablets containing 16 to 48 mg, most preferably 24 to 32 mg, of betahistine dihydrochloride.

The percentage of fatty compound in the tablet ranges from 2 to 40% by weight, preferably from 5 to 15% by weight, based on the weight of the tablet.

The percentage of hydrophilic polymer ranges from 5 to 50%, preferably from 10 to 40%, based on the weight of the tablet.

The invention also relates to multi-layer tablets, preferably double layer tablets, in which at least one layer is a controlled-release one and the other is a prompt-release one.

The tablets according to the invention can be prepared with a process comprising the following steps:
a) subjecting betahistine and the fatty compound to melt-granulation;
b) mixing the granulate from step a) with a hydrophilic compound and with suitable excipients;
c) subjecting to compression the mixture from step b).

The melt-granulation step is carried out heating the mixture above the melting point of the fatty compound in a fluidized bed, in a static oven or in a conventional granulation device.

According to a preferred embodiment of the present invention, the above mentioned process comprises the further step of subjecting the mixture from b) to wet- or dry- granulation before the compression step c).

Tablets can optionally be coated in order to provide a better protection of the active ingredient or to attain further modifications of the release characteristics.

The release characteristics of the composition can be varied adjusting the ratio of fatty compound to hydrophilic polymer.

The in vitro release of the active ingredient can range for example from 6-8 to 24 hours.

The compositions according to the invention can therefore be administered twice or even once a day, depending on the therapeutical requirements to fulfil.

The invention will be further described by means of the following non-limiting examples.

### Example 1

Each tablet contains:
- Betahistine dihydrochloride 32.0 mg
- Stearic acid 22.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg

A melt-granulation process is carried out with a high speed granulator, mixing betahistine and stearic acid. The resulting product is mixed with hydroxypropyl cellulose and talc and wet-granulated with a polyvinylpyrrolidone aqueous solution. The resulting granulate is compressed after addition of silica and glyceryl behenate.

The in vitro release profile is reported in the table hereinbelow.

| **TIME (hours)** | **% RELEASED** |
|---|---|
| 1 | 41.7 |
| 2 | 59.1 |
| 3 | 72.0 |
| 4 | 80.4 |
| 5 | 85.5 |
| 6 | 88.8 |
| 7 | 91.0 |
| 8 | 92.4 |

### Example 2:

Each tablet contains:
- Betahistine dihydrochloride 32.0 mg
- Glyceryl Behenate 22.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg

The preparation procedure is the same as in example 1, using in the melt-granulation the first aliquot of glyceryl behenate (22 mg) in place of stearic acid.

The in vitro release profile is reported in the table hereinbelow.

| **TIME (hours)** | **% RELEASED** |
|---|---|
| 1 | 48.9 |
| 2 | 66.6 |
| 3 | 78.7 |
| 4 | 87.3 |
| 5 | 93.1 |
| 6 | 96.9 |
| 7 | 99.5 |
| 8 | 101.3 |

### Example 3:

Each tablet contains:
- Betahistine dihydrochloride 32.0 mg
- Cetyl alcohol 22.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg

The preparation procedure is the same as in example 1, using in the melt-granulation cetyl alcohol instead of stearic acid.

The in vitro release profile is reported in the table hereinbelow.

| **TIME (hours)** | **% RELEASED** |
|---|---|
| 1 | 49.0 |
| 2 | 67.0 |
| 3 | 79.5 |
| 4 | 88.0 |
| 5 | 93.5 |
| 6 | 97.2 |
| 7 | 99.6 |
| 8 | 101.1 |

### Example 4:

Each tablet contains:
- Betahistine dihydrochloride 32.0 mg
- Cetyl alcohol 32.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg

The preparation procedure is the same as in example 3.

The in vitro release profile is reported in the table hereinbelow.

| **TIME (hours)** | **% RELEASED** |
|---|---|
| 1 | 44.4 |
| 2 | 61.6 |
| 3 | 74.5 |
| 4 | 84.1 |
| 5 | 91.0 |
| 6 | 95.8 |
| 7 | 99.0 |
| 8 | 101.2 |

### Comparative Example 5:

Each tablet contains:
- Betahistine dihydrochloride 32.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg

The preparation procedure involves no melt-granulation step since the fatty compound has been omitted.

The in vitro release profile is reported in the table hereinbelow.

| **TIME (hours)** | **% RELEASED** |
|---|---|
| 1 | 55.0 |
| 2 | 75.0 |
| 3 | 88.0 |
| 4 | 96.7 |
| 5 | 101.2 |

### Comparative Example 6:

Each tablet contains:
- Betahistine dihydrochloride 32.0 mg
- Cetyl alcohol 22.0 mg
- Talc 10.0 mg

The preparation procedure involves a melt-granulation step of betahistine and cetyl alcohol; the talc acts as a lubricant.

The in vitro release profile is reported in the table hereinbelow.

| **TIME (hours)** | **% RELEASED** |
|---|---|
| 1 | 89.1 |
| 2 | 101.2 |

### Comparative Example 7:

Each tablet contains:
- Betahistine dihydrochloride 32.0 mg
- Glyceryl Behenate 22.0 mg
- Talc 14.0 mg

The preparation procedure is the same as in example 6.

The in vitro release profile is reported in the table hereinbelow.

| **TIME (hours)** | **% RELEASED** |
|---|---|
| 1 | 79.0 |
| 2 | 92.3 |
| 3 | 98.2 |
| 4 | 100.5 |

### Comparative Example 8:

Each tablet contains:
- Betahistine dihydrochloride 32.0 mg
- Low viscosity hydroxypropyl cellulose 30.0 mg
- High viscosity hydroxypropyl cellulose 90.0 mg
- Polyvinylpyrrolidone 4.0 mg
- Talc 32.0 mg
- Colloidal silica 16.0 mg
- Glyceryl Behenate 12.0 mg

The preparation procedure involves wet-granulation followed by mixing with lubricants, then compression.

The in vitro release profile is reported in the table hereinbelow.

| **TIME (hours)** | **% RELEASED** |
|---|---|
| 1 | 84.3 |
| 2 | 101.5 |

### Comparative Example 9:

Each tablet contains:
- Betahistine dihydrochloride 32.0 mg
- Low viscosity hydroxypropyl cellulose 60.0 mg
- High viscosity hydroxyprcpyl cellulose 60.0 mg
- Polyvinylpyrrolidone 4.0 mg
- Talc 32.0 mg
- Colloidal silica 16.0 mg
- Glyceryl Behenate 12.0 mg

The preparation procedure is the same as in example 8.

The in vitro release profile is reported in the table hereinbelow.

| **TIME (hours)** | **% RELEASED** |
|---|---|
| 1 | 55.0 |
| 2 | 75.0 |
| 3 | 88.0 |
| 4 | 96.7 |
| 5 | 101.2 |

### Comparative Example 10:

Each tablet contains:
- Betahistine dihydrochloride 32.0 mg
- Ethylcellulose 168 mg
- Hydrogenated castor oil 15.0 mg
- Colloidal silica 5.0 mg

The preparation procedure involves dry-granulation and compression.

The in vitro release profile is reported in the table hereinbelow.

| **TIME (hours)** | **% RELEASED** |
|---|---|
| 1 | 62.2 |
| 2 | 81.1 |
| 3 | 91.8 |
| 4 | 98.2 |
| 5 | 102.1 |

### Example 11:

Each tablet contains:
- Betahiatine dihydrochloride 32.0 mg
- Ethylcellulose 152.0 mg
- Stearic acid 22.0 mg
- Hydrogenated castor oil 15.0 mg
- Colloidal silica 5.0 mg

The preparation procedure is the same as in example 10.

The is vitro release profile is reported in the table hereinbelow.

| **TIME (hours)** | **% RELEASED** |
|---|---|
| 1 | 30.2 |
| 2 | 39.8 |
| 3 | 46.9 |
| 4 | 52.7 |
| 5 | 57.7 |
| 6 | 62.0 |
| 7 | 65.8 |
| 8 | 68.3 |

### Example 12:

Each tablet contains:
- Betahistine dihydrochloride 32.0 mg
- Ethylcellulose 152.0 mg
- Stearic acid 22.0 mg
- Mannitol 10.0 mg
- Hydrogenated castor oil 15.0 mg
- Colloidal silica 10.0 mg

The preparation procedure is the same as in example 10.

The in vitro release profile is reported in the table hereinbelow.

| **TIME (hours)** | **% RELEASED** |
|---|---|
| 1 | 23.9 |
| 2 | 35.1 |
| 3 | 43.3 |
| 4 | 49.6 |
| 5 | 54.4 |
| 6 | 58.1 |
| 7 | 60.8 |
| 8 | 62.9 |

### Example 13:

Each tablet contains:
- Betahistine dihydrochloride 32.0 mg
- Cetyl alcohol 22.0 mg
- Hydroxypropyl cellulose 122.0 mg
- Polyvinylpyrrolidone 4.0 mg
- Talc 44.0 mg
- Colloidal silica 16.0 mg
- Glyceryl Behenate 12.0 mg
- Hydroxypropyl methylcellulose 7.5 mg
- Lactose monohydrate 3.5 mg
- Macrogol 4000 2.5 mg
- Titanium dioxide 1.5 mg

The tablets were obtained by coating the tablets according to Example 3, using the last four components listed.

The in vitro release profile is reported in the table hereinbelow:

| **TIME (hours)** | **% RELEASED** |
|---|---|
| 1 | 44.5 |
| 4 | 84.7 |
| 8 | 99.8 |

### Example 14:

Each tablet contains:
- Betahistine dihydrochloride 32.0 mg
- Cetyl alcohol 22.0 mg
- Hydroxypropyl cellulose 120.0 mg
- Polyvinylpyrrolidone 4.0 mg
- Talc 44.0 mg
- Colloidal silica 16.0 mg
- Glyceryl Behenate 12.0 mg
- Ethylcellulose 5.0 mg
- Dibutylsebacate 1.0 mg
- Lactose 4.0 mg
- Talc 3.0 mg
- Titanium dioxide 2.0 mg

The tablets were obtained coating the tablets according to Example 3 with use of the last five components listed.

| **TIME (hours)** | **% RELEASED** |
|---|---|
| 1 | 38.3 |
| 4 | 76.4 |
| 8 | 98.7 |

## Claims

1. A controlled-release tablet obtainable by:
a) melt granulating betahistine or a pharmaceutically acceptable salt thereof, with at least one fatty compound;
b) mixing the granulate from step a) with at least one hydrophilic polymer capable of adsorbing water and with suitable excipients, and
c) subjecting to compression the mixture from step b).

2. A controlled-release tablet as claimed in claim 1, in which said at least one fatty compound is selected from the group consisting of fatty acids, long-chain fatty acid triglycerids, waxes, vegetable or mineral oils, high molecular alcohols or glycols, the esters and ethers thereof.

3. A controlled-release tablet according to claims 1 or 2, in which said at least one fatty compound has melting point ranging from 30 to 140°C.

4. A controlled-release tablet according to any of claims 1 - 3, in which said at least one hydrophilic polymer is selected from the group consisting of acrylic acid polymers or co-polymers, polyethylene glycols, alginates, cellulose ethers and esters.

5. A controlled-release tablet as claimed in claim 4, in which said at least one hydrophilic polymer is hydroxypropyl cellulose.

6. A controlled-release tablet according to any one of the above claims, in which the percentage of said at least one fatty compound ranges from 2 to 40% by weight based on the weight of the tablet.

7. A controlled-release tablet according to any one of the above claims, in which the percentage of said at least one hydrophilic polymer ranges from 5 to 50% by weight based on the weight of the tablet.

8. A controlled-release tablet according to any one of the above claims, containing an amount of active ingredient equivalent to 8 - 64 mg of betahistine dihydrochloride.

9. A controlled-release tablet as claimed in claim 8, containing 16 to 48 mg, preferably 24 to 32 mg, of betahistine dihydrochloride.

## Patentansprüche

1. Tablette mit kontrollierter Freisetzung, erhältlich durch:
a) Schmeizgranulation von Betahistin oder einem pharmazeutisch verträglichen Salz davon mit mindestens einer Fettverbindung;
b) Mischen des Granulates aus Schritt a) mit mindestens einem hydrophilen Polymer, das fähig ist, Wasser zu adsorbieren, und geeigneten Exzipienten, und
c) Unterwerfen der Mischung aus Schritt b) der Komprimierung.

2. Tablette mit kontrollierter Frelsetzung nach Anspruch 1, worin die genannte, mindestens eine Fettverbindung ausgewählt wird aus der Gruppe, die besteht aus: Fettsäuren, langkettigen Fettsäuretriglyceriden, Wachsen, pflanzlichen Ölen oder Mineralölen, hochmolekularen Alkoholen oder Glykolen, oder deren Ester und Ether.

3. Tablette mit kontrollierter Freisetzung nach Ansprüchen 1 oder 2, worin die genannte, mindestens eine Fettverbindung einen Schmelzpunkt im Bereich von 30 bis 140°C aufweist.

4. Tablette mit kontrollierter Frelsetzung nach irgend einem der Ansprüche 1 - 3, worin das genannte, mindestens eine hydrophlle Polymer ausgewählt wird aus der Gruppe, die besteht aus: Acrylsäurepolymeren oder Copolymeren, Polyethylenglykolen, Alginaten, Celluloseethern und -estern.

5. Tablette mit kontrollierter Freisetzung nach Anspruch 4, worin das genannte mindestens eine hydrophlle Polymer Hydroxypropylcellulose ist.

6. Tablette mit kontrollierter Freisetzung nach irgend einem der obigen Ansprüche, worin der Prozentsatz der mindestens einen Fettverbindung im Bereich von 2 bis 40 Gew.-%, bezogen auf das Gewicht der Tablette, liegt.

7. Tablette mit kontrollierter Freisetzung nach irgend einem der obigen Ansprüche, worin der Prozentsatz des genannten mindestens einem hydrophilen Polymers im Bereich von 5 bis 50 Gew.-%, bezogen auf das Gewlcht der Tablette, liegt.

8. Tablette mit kontrollierter Freisetzung nach irgend einem der obigen Ansprüche, die den aktiven Bestandteil in einer Menge enthält, die äquivalent zu 8 - 64 mg Betahistin-dihydrochlorid ist.

9. Tablette mit kontrollierter Freisetzung nach Anspruch 8, die 16 bis 48 mg, bevorzugt 24 bis 32 mg Betahistin-dihydrochiorid enthält.

## Revendications

1. Comprimé à libération régulée, pouvant être obtenu :
a) en granulant à l'état fondu de la bétahistine ou un sel pharmaceutiquement acceptable de celle-ci, avec au moins un composé gras ;
b) en mélangeant le granulat de l'étape a) avec au moins un polymère hydrophile apte à adsorber de l'eau et avec des excipients appropriés, et
c) en soumettant à compression le mélange de l'étape b).

2. Comprimé à libération régulée selon la revendication 1, dans lequel ledit au moins un composé gras est choisi dans le groupe constitué par les acides gras, les triglycérides d'acide gras à chaîne longue, les cires, les huiles végétales ou minérales, les alcools ou glycols de haut poids moléculaire, les esters et éthers de ceux-ci.

3. Comprimé à libération régulée selon les revendications 1 ou 2, dans lequel ledit au moins un composé gras a un point de fusion allant de 30 à 140 °C.

4. Comprimé à libération régulée selon l'une quelconque des revendications 1-3, dans lequel ledit au moins un polymère hydrophile est choisi dans le groupe constitué par les polymères ou copolymères de l'acide acrylique, les polyéthylèneglycols, les alginates, les éthers et esters de cellulose.

5. Comprimé à libération régulée selon la revendication 4, dans lequel ledit au moins un polymère hydrophile est l'hydroxypropylcellulose.

6. Comprimé à libération régulée selon l'une quelconque des revendications ci-dessus, dans lequel le pourcentage dudit au moins un composé gras va de 2 à 40 % en poids par rapport au poids du comprimé.

7. Comprimé à libération régulée selon l'une quelconque des revendications ci-dessus, dans lequel le pourcentage dudit au moins un polymère hydrophile va de 5 à 50 % en poids par rapport au poids du comprimé.

8. Comprimé à libération régulée selon l'une quelconque des revendications ci-dessus, contenant une quantité d'ingrédient actif équivalant à 8-64 mg de dichlorhydrate de bétahistine.

9. Comprimé à libération régulée selon la revendication 8, contenant 16 à 48 mg, de préférence 24 à 32 mg, de dichlorhydrate de bétahistine.
